# EUROPEAN PATENT SPECIFICATION

(11) **EP 2 245 977 B1**
(45) Date of publication and mention of the grant of the patent: **05.03.2014**
(21) Application number: 10003717.5
(22) Date of filing: 06.04.2010
(51) Int. Cl.: A61B 1/00

(54) **Scopes heating device**
Bereichsheizgerät
Dispositif de chauffage d'objets

(30) Priority: 27.04.2009 TW 98207044 U
(43) Date of publication of application: 03.11.2010
(73) Proprietor: Wang, Wei-Hsun, Yung-Ho City Taipei County 234 (TW)
(72) Inventor: Wang, Ching-Chuan, Yung-Ho City Taipei County 234 (TW)
(74) Representative: Strehl Schübel-Hopf & Partner

(56) References cited:
- WO-A1-2005/044159
- DE-U1-202007 006 946
- US-A- 4 379 448
- US-A1- 2004 267 094
- US-A1- 2009 247 832

## Description

### 1. Field of the Invention:

The present invention relates to means for pre-heating scopes and more particularly, to a scopes heating device based on the technical features of US5,205,278. The scopes heating device uses a fixation device to hold the bag in the folded condition to pre-heat scopes efficiently.

### 2. Description of the Related Art:

Heat storage/release devices are known for years for use to keep the body warm or for the purpose of cold compress. There are many methods to prepare heat storage/release devices. Conventionally, a water bladder may be used to hold hot water for keeping the body warm. There is also known a hot compress bag that comprises a bag body filled with a fluid and sealed, and a metal actuator strip mounted in the bag body and operable to actuate the fluid, causing a chemical reaction and release of heat. During heat producing and releasing procedure, the hot compress bag provides heat energy for hot compress application. US5,205,278, issued to the present inventor, discloses a similar design, entitled "Heat storage/release device for hot/cold compress application", which comprises a bag, which defines therein a sealed accommodation chamber, a heat storage/release material, which is accommodated in the accommodation chamber of the bag and adapted for storing/releasing heat energy, and flexible members fastened to the bag at two opposite sides relative to the accommodation chamber and bendable to hold the bag in a predetermined shape and to secure the bag to a person's face.

Further, following fast development of technology, many scopes have been created and are intensively used to look inside a body cavity or organ. Before application, the scopes must be pre-heated, performing primary sterilization and reducing patient's discomfort.

A heating bag according to the preamble of claim 1 is known from W02005/044159. The bag is partitioned in several chambers and is provided with fastening means. Sodium acetate is stored in the bag; its crystallization generates heat.

A similar heating pad suitable for warming baby bottles is disclosed in DE202007006946.

A heater for endoscopes is described in US2004267094. It comprises a pad for wrapping around an instrument, including one or more substances operative to generate heat through an exothermic reaction; an activation disc is located around the periphery of the pad to provide for convenient user access. A housing contains the pad in sleeve form. The pad may be partitioned to be easier accomodated in the housing.

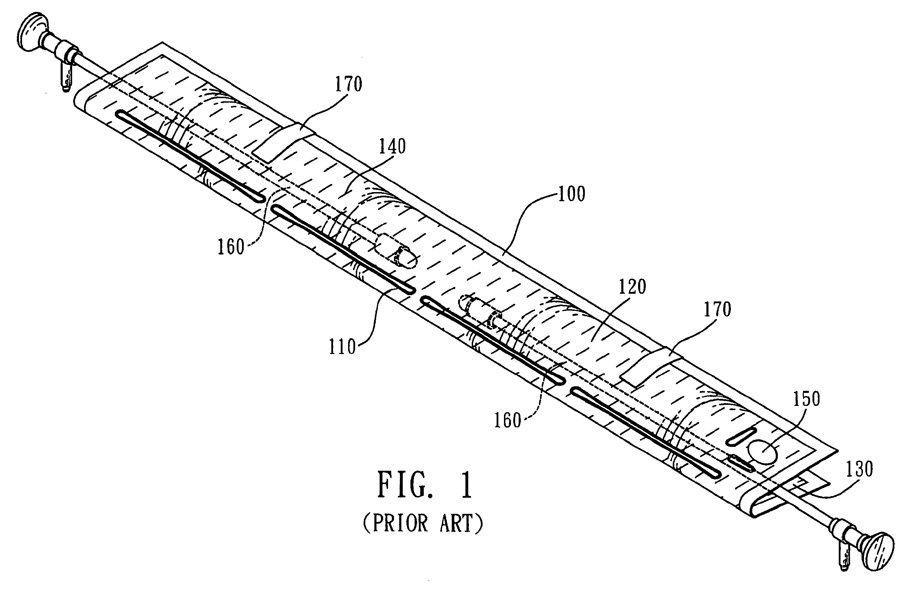

Nowadays, steam heaters and heat storage/release devices are commonly used for pre-heating scopes. FIG. 1 illustrates scopes set in a prior art scope heating device. According to this prior design, the scope heating device comprises a bag **100** that has at least one seal line **110** that divides the inside space of the bag into a first space **120** at one side and a second space **130** at the opposite side, a heat storage/release material **140** mounted inside the bag **100,** and an actuator **150** that is bendable to generate oscillation waves for causing the heat storage/release material **140** to produce a temperature rise and then to release heat so as to pre-heat the scopes **160** that are respectively attached to the first space **120** and the second space **130.**

The aforesaid prior art scope heating device is still not satisfactory in function because of the following drawbacks:
1. During application, the scope heating device is folded up, and two scopes **160** to be pre-heated are respectively set in the first space **120** and the second space **130,** and then an adhesive tape **170** or towel (not shown) is used to wrap the folded scope heating device. This application procedure is complicated. Further, residual substance will be left on the bag **100** after removal of the adhesive tape **170,** causing contamination and resulting in a bad looking.
2. The bag **100** uses longitudinal seal lines **110** to divide the first space **120** and the second space **130.** When the bag **100** is folded up, the scopes **160** are not fully kept in contact with the corresponding lateral side of the first space **120** or the second space **130,** affecting scope heating velocity.

Therefore, there is need for a scopes heating device that eliminates the aforesaid drawbacks.

### SUMMARY OF THE INVENTION

The present invention has been accomplished under the circumstances in view. It is one object of the present invention to provide a scopes heating device, which uses a fixation device to hold scopes in place for pre-heating efficiently.

It is another object of the present invention to provide a scopes heating device, which has at least one first seal line and at least one second seal line formed in the heat storage/release material accommodation bag thereof to divide the bag into a first chamber, a second chamber and a third chamber so that the scopes to be heated are kept in fully contact with the first chamber, the second chamber and the third chamber for pre-heating efficiently.

The invention is defined in the independent claim 1. Preferred embodiments are defined in the dependent claims.

### BRIEF DESCRIPTION OF THE DRAWINGS

FIG. 1 is a schematic drawing showing two scopes set in a folded scopes heating device according to the prior art.
FIG. 2 is an extended view of a scopes heating device according to the present invention.
FIG. 3 is a schematic drawing of the present invention, showing the scopes heating device folded up and scopes set in the folded scopes heating device.
FIG. 4 is an extended view of an alternate form of the scopes heating device according to a preferred embodiment.
FIG. 5 is a schematic drawing of the alternate form of the present invention, showing the scopes heating device folded up and scopes set in the folded scopes heating device.

### DETAILED DESCRIPTION OF THE PREFERRED EMBODIMENT

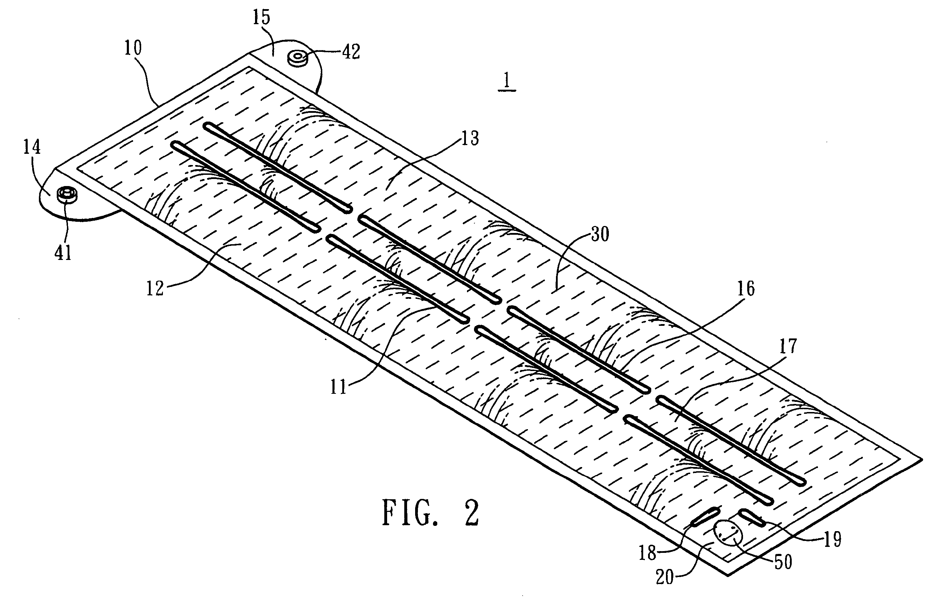

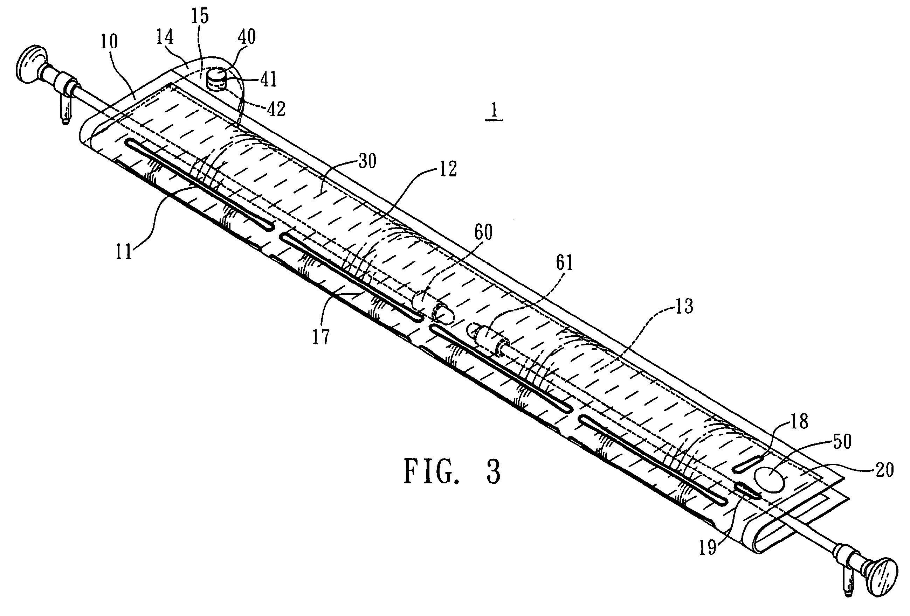

Referring to FIGS. 2 and 3, a scopes heating device **1** in accordance with the present invention is shown comprising a bag **10,** a heat storage/release material **30,** a fixation device **40** and an actuator **50.**

The bag **10** has a predetermined shape, for example, but not limited to, rectangular shape. According to this embodiment, the bag **10** has at least one, for example, but not limited to, four first seal lines **11** longitudinally aligned in line and dividing the inside space of the bag into a first chamber **12** at one side and a second chamber **13** at an opposite side for accommodating the heat storage/release material **30.** The bag **10** is made of, but not limited to, polymeric material. Further, the bag **10** is preferably a transparent bag. For the purpose of being reusable, the material of the bag **10** has durable and extensible characteristics and a certain thickness. During fabrication, high frequency sealing technique or any other suitable sealing technique is employed to seal the periphery of the bag **10.** This bag fabrication method is of the known art and not within the scope of the claims of the present invention, and therefore no further detailed description in this regard is necessary.

The heat storage/release material **30** has the power of storing/releasing heat energy, and is accommodated in the bag **10.** The heat storage/release material 30 can be pure water, saturated sodium acetate solution, or any suitable known fluid that releases heat gradually after having been heated. Further, the heat storage/release material can be stored in a refrigerator (not shown).

The fixation device **40** is mounted on the bag **10** for securing the first chamber **12** and the second chamber **13** in a stacked manner when the bag **10** is folded up. As illustrated, the fixation device **40** comprises a first fastening member **41** and a second fastening member **42.** The first fastening member **41** is, for example, a male fastening member provided at the top side of the first chamber **12.** The second fastening member **42** is, for example, a female fastening member provided at the top side of the second chamber 13 and connectable to the first fastening member **41.**

The bag **10** further has two flaps **14** and **15** respectively extended from two sides of one end thereof for carrying the first fastening member **41** and the second fastening member **42** respectively. The flaps **14** and **15** are fixedly fastened to the border of the bag **10** by means of, for example, but not limited to, a high-frequency heat sealing technique.

The bag **10** further has at least one, for example, but not limited to, four second seal lines **16** longitudinally aligned in line and disposed in a parallel manner relative to the first seal lines **11,** thereby defining a third chamber **17** between the first seal lines **11** and the second seal lines **16.** Further, the heat storage/release material **30** is flowing among the first chamber **12,** the second chamber **13** and the third chamber **17.** Further, the first seal lines **11** and the second seal lines **16** can be produced by means of a high-frequency heat sealing technique.

The bag **10** further has a third seal line **18** and a fourth seal line **19** disposed at right angles around one corner thereof remote from the flaps **14** and **15,** defining a fourth chamber **20** for accommodating the actuator **50.** The third seal line **18** and the fourth seal line **19** are made by means of a high-frequency heat sealing technique.

The actuator **50** is mounted in the fourth chamber **20** of the bag **10.** Further, the actuator **50** is, for example, but not limited to, a metal strip that is bendable to generate oscillation wavess for causing the heat storage/release material **30** to produce a temperature rise and then to release heat. With respect to this temperature rise and heat release function, please refer to the description of US5,025,278.

Referring to FIG. 3, during application of the scopes heating device **1,** attach the two scopes **60** and **61** to be warmed up to the bag **10** corresponding to the third chamber **17,** and then fold up the bag **10** to let the two scopes **60** and **61** be surrounded by the third chamber **17,** the first chamber **12** and the second chamber **13,** and then fasten the first fastening member **41** at the first chamber **12** to the second fastening member **42** at the second chamber **13** to secure the bag **10** in the folded condition and to keep the two scopes **60** and 61 in fully contact with the third chamber **17,** the first chamber **12** and the second chamber **13,** and finally bend the actuator **50** to generate oscillation wavess that cause the heat storage/release material **30** to produce a temperature rise and to further release heat, thereby pre-heating the scopes **60** and **61.** Thus, the scopes heating device **1** improves the drawbacks of the prior art design.

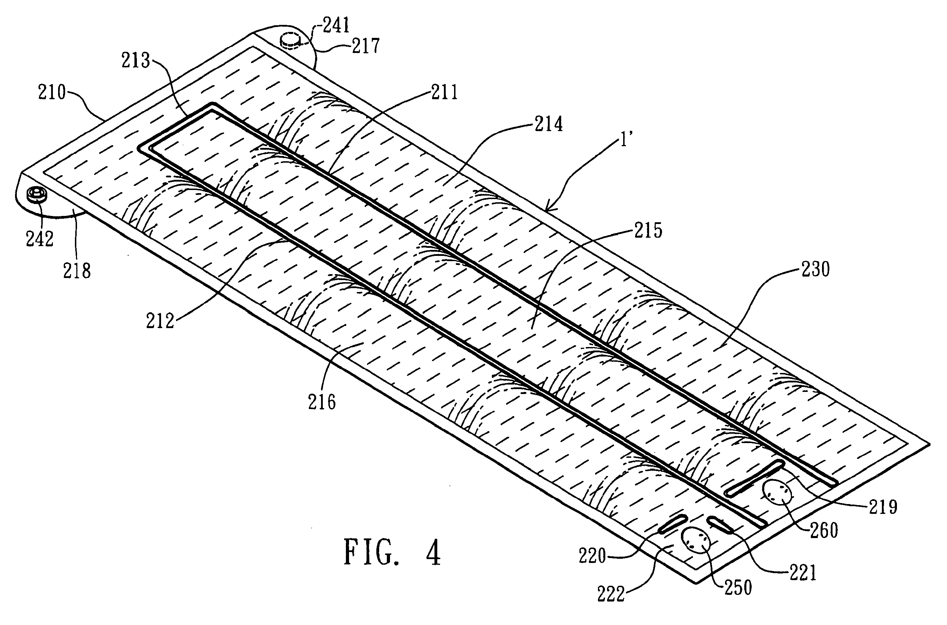

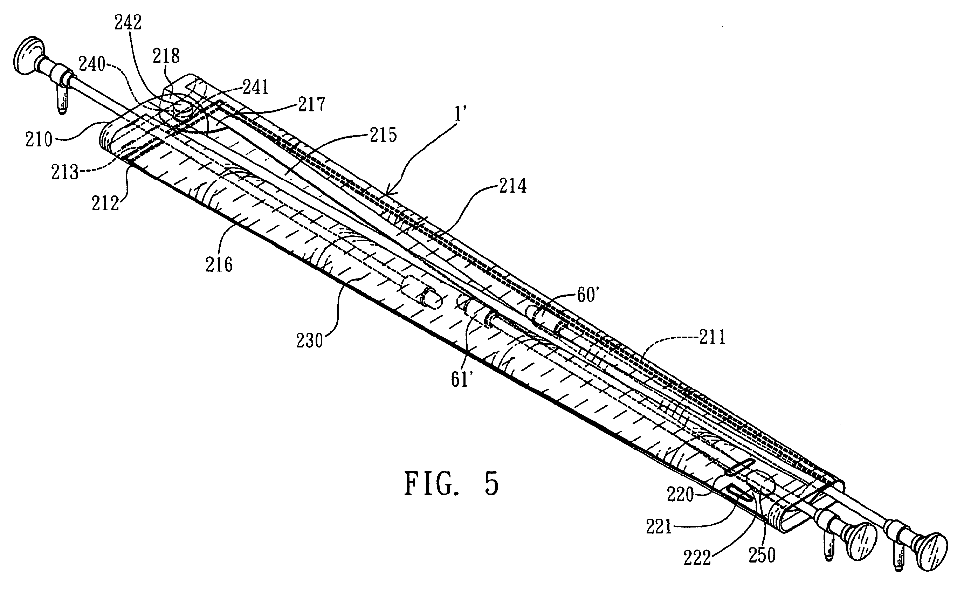

FIGS. 4 and 5 show a preferred embodiment of the present invention. According to this alternate form, the scopes heating device **1'** comprises a bag **210,** a heat storage/release material **230,** a fixation device **240,** a first actuator **250** and a second actuator **260.**

The bag **210** has a predetermined shape, for example, but not limited to, rectangular shape. According to this alternate form, the bag **210** has a first seal line **211** and a second seal line **212** longitudinally arranged in parallel, a third seal line **213** transversely connected between one end of the first seal line **211** and the corresponding end of the second seal line **212,** a first chamber **214** defined therein at one lateral side relative to the first seal line **211,** a second chamber **215** defined between the first seal line **211** and the second seal line **212,** and a third chamber **216** defined therein at one lateral side relative to the second seal line **212.** The first chamber **214,** the second chamber **215** and the third chamber **216** accommodate the heat storage/release material **230.** The first chamber **214** and the third chamber **216** are in fluid communication with each other and isolated from the second chamber **215.** The bag **210** is made of, but not limited to, polymeric material. Further, the bag **210** is preferably a transparent bag. For the purpose of being reusable, the material of the bag **210** has durable and extensible characteristics and a certain thickness. During fabrication, high frequency sealing technique or any other suitable sealing technique is employed to seal the periphery of the bag **210.** This bag fabrication method is of the known art and not within the scope of the claims of the present invention, and therefore no further detailed description in this regard is necessary.

The heat storage/release material **230** has the power of storing/releasing heat energy, and is accommodated in the chambers **214∼216** of the bag **210.** The heat storage/release material **230** can be pure water, saturated sodium acetate solution, or any suitable known fluid that releases heat gradually after having been heated. Further, the heat storage/release material can be stored in a refrigerator (not shown).

The fixation device **240** is mounted on the bag **210** for securing the first chamber **214** and the third chamber **216** in a stacked manner when the bag **210** is folded up. As illustrated, the fixation device **240** comprises a first fastening member **241** and a second fastening member **242.** The first fastening member **241** is, for example, a male fastening member provided at the top side of the first chamber **214.** The second fastening member **242** is, for example, a female fastening member provided at the top side of the third chamber **216** and connectable to the first fastening member **241.**

The bag **210** further has two flaps **217** and **218** respectively extended from two sides of one end thereof for carrying the first fastening member **241** and the second fastening member **242** respectively. The flaps **217** and **218** are fixedly fastened to the border of the bag **210** by means of, for example, but not limited to, a high-frequency heat sealing technique.

The bag **210** further has a fourth seal line **219** spaced between the spaced between the first seal line **211** and the second seal line **212** near the other end remote from the third seal line **213** defining a space for accommodating the second actuator **260.** The fourth seal line **219** is made by means of a high-frequency heat sealing technique.

The bag **210** further has a fifth seal line **220** and a sixth seal line **221** disposed at right angles around one corner of the third chamber **216,** defining a fourth chamber **222** for accommodating the first actuator **250.** The fifth seal line **220** and a sixth seal line **221** are made by means of a high-frequency heat sealing technique.

The first actuator **250** is accommodated in the fourth chamber **222** inside the bag **210.** Further, the first actuator **250** is, for example, but not limited to, a metal strip that is bendable to generate oscillation waves for causing the heat storage/release material **230** to produce a temperature rise and then to release heat. With respect to this temperature rise and heat release function, please refer to the description of US5,205,278.

The second actuator **260** is accommodated in one end of the second chamber **215** at one side relative to the fourth seal line **219.** Further, the second actuator **260** is, for example, but not limited to, a metal strip that is bendable to generate oscillation waves for causing the heat storage/release material **230** to produce a temperature rise and then to release heat. With respect to this temperature rise and heat release function, please refer to the description of US5,205,278.

Referring to FIG. 5, during application of the scopes heating device **1',** attach a first scope **60'** to be warmed up to the bag **210** corresponding to the second chamber **215,** and then fold up one lateral side of the bag **210** to have the folded part of the bag **210** be covered on the first scope **60',** and then attach a second scope **61'** to be warmed up to the bag **210** corresponding to the first chamber **214,** and then fold up the other lateral side of the bag **210** to have the part of the bag **210** corresponding to the third chamber **216** be covered on the second scope **61',** and then fasten the first fastening member **241** at the first chamber **214** to the second fastening member **242** at the third chamber **216** to secure the bag **210** in the folded condition and to keep the two scopes **60'** and **61'** in fully contact with the third chamber **216,** the first chamber **214** and the second chamber **215,** and finally bend the first actuator **250** to generate oscillation waves that cause the heat storage/release material **230** to produce a temperature rise and to further release heat, thereby pre-heating the scopes **60'** and **61'.** If the operation time must be extended, bend the second actuator **260** to generate oscillation waves that cause the heat storage/release material **230** to produce a temperature rise and to further release heat, thereby continuously warming up the scopes **60'** and **61'.** Thus, the scopes heating device **1'** improves the drawbacks of the prior art design.

As stated above, the scopes heating device is provided with a fixation that holds the bag of the scopes heating device in a folded condition to keep scopes in fully contact with the bag for pre-heating positively. Further, the bag of the scopes heating device has at least one first seal line and at least one second seal line to divide the inside space of the bag into a first chamber, a second chamber and a third chamber. Thus, when the bag is folded up, these three chambers are kept in contact with the scopes to be pre-heated, enabling the scopes to be quickly pre-heated. Therefore, the invention effectively improves the drawbacks of the prior art design.

A prototype of scopes heating device has been constructed with the features of FIGS. 2 and 3. The scopes heating device functions smoothly to provide all of the features disclosed earlier.

## Claims

1. A scopes heating device for pre-heating scopes, comprising:
a bag (10), said bag (10) having at least one first seal line (11) that divides said bag (10) into a first chamber (12) at one side relative to said at least one first seal line (11) and a second chamber (13) at an opposite side relative to said at least one first seal line (11);
a heat storage/release material (30) accommodated in said bag (10) and adapted for storing/releasing heat energy;
a fixation device (40) mounted on said bag (10) and adapted to secure said bag (10) in a folded condition where said first chamber (12) and said second chamber (13) are stacked up, wherein said fixation device (40) comprises:
a first fastening member (41) provided at said bag (10) corresponding to said first chamber (12), wherein said first fastening member (41) is a male fastening member; and
a second fastening member (42) provided at said bag (10) corresponding to said second chamber (13) and fastenable to said first fastening member (41), wherein said second fastening member (42) is a female fastening member; and
an actuator (50) formed of a metal strip and mounted in said bag (10) and bendable for causing said heat storage/release material (30) to produce a temperature rise and then to release heat for pre-heating scopes being set between said first chamber (12) and said second chamber (13) after said bag (10) having been folded up and said actuator (50) is bendable to generate oscillation waves for causing said heat storage/release material (30) to produce a temperature rise.
wherein said bag (10) comprises a first flap (14) and a second flap (15) respectively protruded from two opposite sides of one end thereof; said first fastening member (41) and said second fastening member (42) are respectively located on said first flap (14) and said second flap (15),
**characterized in that**
said at least one seal line (11) is Longitudinally located on a middle part of said bag (10).

2. The scopes heating device as claimed in claim 1, wherein said heat storage/release material (30) is saturated sodium acetate solution, and said bag (10) is made of a polymeric material.

3. The scopes heating device according to claim 2, wherein said bag (10) further comprises at least one second seal line (16) disposed at one lateral side relative to said at least one first seal line (11) and defining a third chamber (17) in fluid communication between said first chamber (12) and said second chamber (13), wherein said at least one first seal line (11), said at least one second seal line (16) and said flaps (14, 15) are made by means of a sealing technique.

4. The scopes heating device as claimed in claim 3, wherein said bag (10) further comprises a third seal line (18) and a fourth seal line (19) arranged at right angles around one corner thereof remote from said fixation device (40) and defining a fourth chamber (20) in said bag (10) for accommodating said actuator (50); said third seal line (18) and said fourth seal line (19) are formed by means of a sealing technique.

5. A scopes heating device for pre-heating scopes according to claim 1,
wherein said bag (210), comprises further a second seal line (212), a third seal line (213), and wherein said first chamber (214) is defined at one side of said first seal line (211) opposite to said second seal line (212), a second chamber (215) is defined between said first seal line (211) and said second seal line (212), a third chamber (216) is defined at one side relative to said second seal line (212) opposite to said first seal line (211);
said fixation device (240) is mounted on said bag (210) and adapted to secure said bag (210) in a folded condition where said first chamber (214) and said third chamber (216) are stacked up;
said actuator (250) is mounted in one of the first chamber (214) and third chamber (216) of said bag (210) **characterized in that** it further comprises
a second actuator (260) formed of a metal strip and mounted in said second chamber (215) of said bag (210) and bendable for causing said heat storage/release material to produce a temperature rise and then to release heat for pre-heating scopes being set between said second chamber (215) and said third chamber (216) after said bag having been folded up.

6. The scopes heating device as claimed in claim 5, wherein said heat storage/release material (230) is saturated sodium acetate solution, said bag (210) is made of a polymeric material, and said first chamber (214) and said third chamber (216) are in fluid communication with each other and isolated from said second chamber (215).

7. The scopes heating device as claimed in claim 5 wherein said fixation device (240) comprises:
a first fastening member (241) provided at said bag (210) corresponding to said first chamber (214), wherein said first fastening member (241) is a male fastening member; and
a second fastening member (242) provided at said bag (210) corresponding to said third chamber (216) and fastenable to said first fastening member (241), wherein said second fastening member (242) is a female fastening member.

8. The scopes heating device as claimed in claim 7, wherein said bag (210) comprises a first flap (217) and a second flap (218) respectively protruded from two opposite sides of one end thereof; said first fastening member (241) and said second fastening member (242) are respectively located on said first flap (217) and said second flap (218).

9. The scopes heating device as claimed in claim 5, wherein said first seal line (211) and said second seal line (212) are longitudinally arranged in parallel; said third seal line (213) is transversely connected between one end of said first seal line (211) and one end of said second seal line (212), said first actuator (250) and second actuator (260) are bendable to generate oscillation waves for causing said heat storage/release material (230) to produce a temperature rise.

10. The scopes heating device as claimed in claim 5, wherein said bag (210) further comprises a fourth seal line (219) transversely disposed in said second chamber (215) and spaced between said first seal line (211) and said second seal line (212) remote from said third seal line (213) and defining a space in said second chamber (215) for accommodating said second actuator (260).

11. The scopes heating device as claimed in claim 8, wherein said first seal line (211), said second seal line (212) and said flaps (217, 218) are made by means of a sealing technique.

12. The scopes heating device as claimed in claim 5, wherein said bag (210) further comprises a fifth seal line (220) and a sixth seal line (221) arranged at right angles around one corner of said third chamber (216) remote from said fixation device (240) and defining a fourth chamber (222) in said bag (210) for accommodating said first actuator (250); said fifth seal line (220) and said sixth seal line (221) are formed by means of a sealing technique.

## Patentansprüche

1. Bereichsheizvorrichtung zum Vorerhitzen von Bereichen, welche umfasst:
eine Tasche (10), wobei die Tasche (10) mindestens eine erste Versiegelungslinie (11), welche die Tasche (10) in eine erste Kammer (12) auf der in Bezug auf die mindestens erste Versiegelungslinie (11) einen Seite und eine zweite Kammer (13) auf einer in Bezug auf die mindestens eine erste Versiegelungslinie (11) gegenüberliegenden Seite aufweist;
ein Wärmespeicherungs-/Freisetzungsmaterial (30), das sich ihn der Tasche (10) befindet und für die Speicherung/Freisetzung von Wärmeenergie angepasst ist;
eine Festhaltevorrichtung (40), die an der Tasche (10) angebracht ist und zur Sicherung der Tasche (10) in einem gefalteten Zustand angepasst ist, wobei die erste Kammer (12) und die zweite Kammer (13) gestapelt sind, wobei die Festhaltevorrichtung (40) umfasst:
ein erstes Befestigungselement (41), das an der Tasche (10) angeordnet ist und mit der ersten Kammer (12) korrespondiert, wobei das erste Festhalteelement (41) ein männliches Festhalteelement ist; und ein zweites Festhalteelement (42), das an der Tasche (10) angeordnet ist, mit der zweiten Kammer (13) korrespondiert und an dem ersten Festhalteelement (41) befestigt werden kann, wobei das zweite Festhalteelement (42) ein weibliches Festhalteelement ist; und
einen Auslöser (50), der aus einem Metallstreifen gebildet ist und in der Tasche (10) angeordnet ist und biegsam ist, um zu bewirken, dass das Wärmespeicherungs-/Freisetzungsmaterial (30) eine Temperaturerhöhung produziert und dann für das Vorerhitzen von Bereichen, die zwischen der ersten Kammer (12) und der zweiten Kammer (13) angeordnet sind, Wärme freisetzt, nachdem die Tasche (10) zusammengelegt worden ist, und wobei der Auslöser (50) biegsam ist, um Oszillationswellen zu erzeugen, um zu bewirken, dass das Wärmespeicherungs-/Freisetzungsmaterial (30) eine Temperaturerhöhung produziert,
wobei die Tasche (10) mindestens eine erste Lasche (14) und eine zweite Lasche (15) aufweist, die jeweils von zwei gegenüberliegenden Seiten an einem Ende der Tasche hervorstehen;
wobei das erste Festhalteelement (41) und das zweite Festhalteelement (42) jeweils auf der ersten Lasche (14) und der zweiten Lasche (15) angeordnet sind,
**dadurch gekennzeichnet, dass**
mindestens eine Versiegelungslinie (11) auf einem Mittelteil der Tasche (10) in Längsrichtung angeordnet ist.

2. Bereichsheizvorrichtung nach Anspruch 1, wobei das Wärmespeicherungs-/Freisetzungsmaterial (30) eine gesättigte Natriumacetatlösung ist, und die Tasche (10) aus einem Polymermaterial besteht.

3. Bereichsheizvorrichtung nach Anspruch 2, wobei die Tasche (10) außerdem mindestens eine zweite Versiegelungslinie (16) aufweist, die in Bezug auf die mindestens eine erste Versiegelungslinie (11) auf einer lateralen Seite angeordnet ist und eine dritte Kammer (17) definiert, wobei zwischen der ersten Kammer (12) und der zweiten Kammer (13) Fluide fließen können, wobei die mindestens eine erste Versiegelungslinie (11), die mindestes eine zweite Versiegelungslinie (16) und die Laschen (14, 15) mit Hilfe einer Versiegelungstechnik gebildet sind.

4. Bereichsheizvorrichtung nach Anspruch 3, wobei die Tasche (10) außerdem eine dritte Versiegelungslinie (18) und eine vierte Versiegelungslinie (19) umfasst, die um eine Ecke der Tasche im rechten Winkel angeordnet sind, wobei die Ecke von der Befestigungsvorrichtung (40) entfernt ist und eine vierte Kammer (20) in der Tasche (10) definiert, um den Auslöser (50) aufzunehmen; wobei die dritte Versiegelungslinie (18) und die vierte Versiegelungslinie (19) mit Hilfe einer Versiegelungstechnik gebildet sind.

5. Bereichsheizvorrichtung zum Vorerhitzen von Bereichen nach Anspruch 1, wobei der Tasche (210) außerdem eine zweite Versiegelungslinie (212), eine dritte Versiegelungslinie (213) aufweist, und wobei die erste Kammer (214) auf einer Seite der ersten Versiegelungslinie (211) gegenüber der zweiten Versiegelungslinie (212) definiert ist, eine zweite Kammer (215) zwischen der ersten Versiegelungslinie (211) und der zweiten Versiegelungslinie (212) definiert ist, eine dritte Kammer (216) an einer in Bezug auf die zweite Versiegelungslinie (212) gegenüber der ersten Versiegelungslinie (211) befindlichen Seite definiert ist; wobei die Befestigungsvorrichtung (240) auf der Tasche (210) angeordnet ist und angepasst ist, um die Tasche (210) in einem gefalteten Zustand zu sichern, wobei die erste Kammer (214) und die dritte Kammer (216) gestapelt sind; wobei der Auslöser (250) in einer der ersten Kammer (214) und dritten Kammer (216) der Tasche (210) angeordnet ist,
**dadurch gekennzeichnet, dass** sie außerdem einen zweiten Auslöser (260), der aus einem Metallstreifen gebildet ist und in der zweiten Kammer (215) der Tasche (210) angeordnet ist und biegsam ist, um das Wärmespeicherungs-/Freisetzungsmaterial zu einem Temperaturanstieg und dann zur Wärmefreisetzung zu veranlassen, um Bereiche zwischen der zweiten Kammer (215) und der dritten Kammer (216) zu erwärmen, nachdem die Tasche aufgefaltet worden ist.

6. Bereichsheizvorrichtung nach Anspruch 5, wobei das Wärmespeicherungs-/Freisetzungsmaterial (230) eine gesättigte Natriumacetatlösung ist, die Tasche (210) aus einem Polymermaterial gebildet ist, und die erste Kammer (214) und die dritte Kammer (216) so angeordnet sind, dass zwischen ihnen Fluide fließen können und sie von der zweiten Kammer (215) isoliert sind.

7. Bereichsheizvorrichtung nach Anspruch 5, wobei die Befestigungsvorrichtung (240) umfasst:
ein erstes Festhalteelement (241), das an der Tasche (210) angeordnet ist und mit der ersten Kammer (214) korrespondiert, wobei das erste Festhalteelement (241) ein männliches Festhalteelement ist; und
ein zweites Festhalteelement (242), das an der Tasche (210) angeordnet ist, mit der dritten Kammer (216) korrespondiert und an das erste Festhalteelement (241) befestigt werden kann, wobei das zweite Festhalteelement (242) ein weibliches Festhalteelement ist.

8. Bereichsheizvorrichtung nach Anspruch 7, wobei die Tasche (210) eine erste Lasche (217) und eine zweite Lasche (218) umfasst, die jeweils von zwei gegenüberliegenden Seiten eines Endes der Taschen hervorstehen; wobei das erste Festhalteelement (241) und das zweite Festhalteelement (242) jeweils auf der ersten Lasche (217) und der zweiten Lasche (218) angeordnet sind.

9. Bereichsheizvorrichtung nach Anspruch 5, wobei die erste Versiegelungslinie (211) und die zweite Versiegelungslinie (212) in Längsrichtung parallel angeordnet sind; die dritte Versiegelungslinie (213) zwischen einem Ende der ersten Versiegelungslinie (211) und einem Ende der zweiten Versiegelungslinie (212) quer angeordnet ist, wobei der erste Auslöser (250) und der zweite Auslöser (260) biegsam sind, so dass Oszillationswellen erzeugt werden, um zu bewirken, dass das Wärmespeicherungs-/Freisetzungsmaterial (230) einen Temperaturanstieg erzeugt.

10. Bereichsheizvorrichtung nach Anspruch 5, wobei die Tasche (210) außerdem eine vierte Versiegelungslinie (219), die in der zweiten Kammer (215) quer angeordnet ist und zwischen der ersten Versiegelungslinie (211) und der zweiten Versiegelungslinie (212) einen Abstand aufweist, wobei diese von der dritten Versiegelungslinie (213) entfernt sind und in der zweiten Kammer (215) zum Aufnehmen des zweiten Auslösers (260) einen Abstand definieren.

11. Bereichsheizvorrichtung nach Anspruch 8, wobei die erste Versiegelungslinie (211), die zweite Versiegelungslinie (212) und die Laschen (217, 218) mit Hilfe einer Versiegelungstechnik hergestellt sind.

12. Bereichsheizvorrichtung nach Anspruch 5, wobei die Tasche (210) außerdem eine fünfte Versiegelungslinie (220) und eine sechste Versiegelungslinie (221) aufweist, die um eine Ecke der dritten Kammer (216), die von der Befestigungsvorrichtung (240) entfernt ist, in einem rechten Winkel angeordnet sind und eine vierte Kammer (222) in der Tasche (210) zur Aufnahme des ersten Auslösers (250) definieren; wobei die fünfte Versiegelungslinie (220) und die sechste Versiegelungslinie (221) mit Hilfe einer Versiegelungsvorrichtung gebildet sind.

## Revendications

1. Dispositif de chauffage d'instruments médicaux pour préchauffer des instruments médicaux, comprenant :
un sac (10), ledit sac (10) comportant au moins une première ligne de scellement (11) qui divise ledit sac (10) en une première chambre (12) d'un côté de ladite au moins une première ligne de scellement (11) et une seconde chambre (13) d'un côté opposé à ladite au moins une première ligne de scellement (11) ;
un matériau de stockage/libération de chaleur (30) logé dans ledit sac (10) et adapté pour stocker/libérer de l'énergie thermique ;
un dispositif de fixation (40) monté sur ledit sac (10) et adapté pour fixer ledit sac (10) dans un état plié où ladite première chambre (12) et ladite seconde chambre (13) sont empilées, dans lequel ledit dispositif de fixation (40) comprend : un premier élément de fixation (41) placé sur ledit sac (10) correspondant à ladite première chambre (12), dans lequel ledit premier élément de fixation (41) est un élément de fixation mâle ; et un second élément de fixation (42) placé sur ledit sac (10) correspondant à ladite seconde chambre (13) et pouvant être fixé sur ledit premier élément de fixation (41), dans lequel ledit second élément de fixation (42) est un élément de fixation femelle ; et
un actionneur (50) formé d'une bande métallique et monté dans ledit sac (10) et pouvant être plié pour faire que ledit matériau de stockage/libération de chaleur (30) produise une élévation de température et pour ensuite libérer de la chaleur pour préchauffer des instruments médicaux placés entre ladite première chambre (12) et ladite seconde chambre (13) après que ledit sac (10) a été plié, et ledit actionneur (50) peut être plié pour générer des ondes d'oscillation pour faire que ledit matériau de stockage/libération de chaleur (30) produise une élévation de température,
dans lequel ledit sac (10) comprend un premier volet (14) et un second volet (15) faisant saillie respectivement depuis deux côtés opposés d'une extrémité de celui-ci ; ledit premier élément de fixation (41) et ledit second élément de fixation (42) sont situés respectivement sur ledit premier volet (14) et ledit second volet (15),
**caractérisé en ce que**
ladite au moins une ligne de scellement (11) est située longitudinalement sur une partie médiane dudit sac (10).

2. Dispositif de chauffage d'instruments médicaux selon la revendication 1, dans lequel ledit matériau de stockage/libération de chaleur (30) est une solution d'acétate de sodium saturée, et ledit sac (10) est fait d'un matériau polymère.

3. Dispositif de chauffage d'instruments médicaux selon la revendication 2, dans lequel ledit sac (10) comprend en outre au moins une seconde ligne de scellement (16) disposée latéralement d'un côté de ladite au moins une première ligne de scellement (11) et définissant une troisième chambre (17) en communication fluide entre ladite première chambre (12) et ladite seconde chambre (13), dans lequel ladite au moins une première ligne de scellement (11), ladite au moins une seconde ligne de scellement (16) et lesdits volets (14, 15) sont fait au moyen d'une technique de scellement.

4. Dispositif de chauffage d'instruments médicaux selon la revendication 3, dans lequel ledit sac (10) comprend en outre une troisième ligne de scellement (18) et une quatrième ligne de scellement (19) agencées à angle droit autour d'un coin de celui-ci à distance dudit dispositif de fixation (40) et définissant une quatrième chambre (20) dans ledit sac (10) pour loger ledit actionneur (50) ; ladite troisième ligne de scellement (18) et ladite quatrième ligne de scellement (19) sont formées au moyen d'une technique de scellement.

5. Dispositif de chauffage d'instruments médicaux pour préchauffer des instruments médicaux selon la revendication 1, dans lequel ledit sac (210) comprend en outre une seconde ligne de scellement (212), une troisième ligne de scellement (213), et dans lequel ladite première chambre (214) est définie d'un côté de ladite première ligne de scellement (211) opposé à ladite seconde ligne de scellement (212), une second chambre (215) est définie entre ladite première ligne de scellement (211) et ladite seconde ligne de scellement (212), une troisième chambre (216) est définie d'un côté de ladite seconde ligne de scellement (212) opposé à ladite première ligne de scellement (211) ;
ledit dispositif de fixation (240) est monté sur ledit sac (210) et adapté pour fixer ledit sac (210) dans un état plié où ladite première chambre (214) et ladite troisième chambre (216) sont empilées ;
ledit actionneur (250) est monté dans l'une de la première chambre (214) et de la troisième chambre (216) dudit sac (210) ;
**caractérisé en ce qu'**il comprend en outre un second actionneur (260) formé d'une bande métallique et monté dans ladite seconde chambre (215) dudit sac (210) et pouvant être plié pour faire que ledit matériau de stockage/libération de chaleur produise une élévation de température et de libérer ensuite de la chaleur pour préchauffer des instruments médicaux placés entre ladite seconde chambre (215) et ladite troisième chambre (216) après que ledit sac a été plié.

6. Dispositif de chauffage d'instruments médicaux selon la revendication 5, dans lequel ledit matériau de stockage/libération de chaleur (230) est une solution d'acétate de sodium saturée, ledit sac (210) est fait d'un matériau polymère, et ladite première chambre (214) et ladite troisième chambre (216) sont en communication fluide ensemble et isolées de ladite seconde chambre (215).

7. Dispositif de chauffage d'instruments médicaux selon la revendication 5, dans lequel ledit dispositif de fixation (240) comprend ;
un premier élément de fixation (241) placé sur ledit sac (210) correspondant à ladite première chambre (214), dans lequel ledit premier élément de fixation (241) est un élément de fixation mâle ; et
un second élément de fixation (242) placé sur ledit sac (210) correspondant à ladite troisième chambre (216) et pouvant être fixé sur ledit premier élément de fixation (241), dans lequel ledit second élément de fixation (242) est un élément de fixation femelle.

8. Dispositif de chauffage d'instruments médicaux selon la revendication 7, dans lequel ledit sac (210) comprend un premier volet (217) et un second volet (218) faisant saillie respectivement depuis deux côtés opposés d'une extrémité de celui-ci ; ledit premier élément de fixation (241) et ledit second élément de fixation (242) sont situés respectivement sur ledit premier volet (217) et ledit second volet (218).

9. Dispositif de chauffage d'instruments médicaux selon la revendication 5, dans lequel ladite première ligne de scellement (211) et ladite seconde ligne de scellement (212) sont agencées longitudinalement en parallèle ; ladite troisième ligne de scellement (213) est reliée transversalement entre une extrémité de ladite première ligne de scellement (211) et une extrémité de ladite seconde ligne de scellement (212), lesdits premier actionneur (250) et second actionneur (260) peuvent être pliés pour générer des ondes d'oscillation pour faire que ledit matériau de stockage/libération de chaleur (230) produise une élévation de température.

10. Dispositif de chauffage d'instruments médicaux selon la revendication 5, dans lequel ledit sac (210) comprend en outre une quatrième ligne de scellement (219) disposée transversalement dans ladite seconde chambre (215) et espacée entre ladite première ligne de scellement (211) et ladite seconde ligne de scellement (212) à distance de ladite troisième ligne de scellement (213) et définissant un espace dans ladite seconde chambre (215) pour loger ledit second actionneur (260).

11. Dispositif de chauffage d'instruments médicaux selon la revendication 8, dans lequel ladite première ligne de scellement (211), ladite seconde ligne de scellement (212) et lesdits volets (217, 218) sont faits au moyen d'une technique de scellement.

12. Dispositif de chauffage d'instruments médicaux selon la revendication 5, dans lequel ledit sac (210) comprend en outre une cinquième ligne de scellement (220) et une sixième ligne de scellement (221) agencées à angle droit autour d'un coin de ladite troisième chambre (216) à distance dudit dispositif de fixation (240) et définissant une quatrième chambre (222) dans ledit sac (210) pour loger ledit premier actionneur (250) ; ladite cinquième ligne de scellement (220) et ladite sixième ligne de scellement (221) sont formées au moyen d'une technique de scellement.
